# EUROPÄISCHE PATENTSCHRIFT

(11) **EP 2 938 204 B1**
(45) Veröffentlichungstag und Bekanntmachung des Hinweises auf die Patenterteilung: **01.03.2017**
(21) Anmeldenummer: 13811552.2
(22) Anmeldetag: 20.12.2013
(51) Int. Cl.: A23J 1/00, A23J 1/14

(54) **VERFAHREN ZUR GEWINNUNG VON WERTPRODUKTEN, INSBESONDERE PROTEINEN, AUS EINEM NATIVEN STOFFGEMENGE**
METHOD FOR OBTAINING VALUABLE PRODUCTS, IN PARTICULAR PROTEINS, FROM A NATIVE MIXTURE OF MATERIALS
PROCEDE PERMETTANT D'OBTENIR DES PRODUITS VALORISABLES, NOTAMMENT DES PROTEINES, A PARTIR D'UNE MELANGE DE MATIERES NATIVES

(30) Priorität: 27.12.2012 DE 102012113100; 18.04.2013 DE 102013103910
(43) Veröffentlichungstag der Anmeldung: 04.11.2015
(73) Patentinhaber: GEA Mechanical Equipment GmbH, 59302 Oelde (DE)
(72) Erfinder: HRUSCHKA, Steffen, 59302 Oelde (DE); BOSZULAK, Wladislawa, 59302 Oelde (DE); ULLMANN, Detlef, 59302 Oelde (DE); RASSENHÖVEL, Jürgen, 59032 Oelde (DE)
(74) Vertreter: Specht, Peter
(86) Internationale Anmeldenummer: PCT/EP2013/077621
(87) Internationale Veröffentlichungsnummer: WO 2014/102176

(56) Entgegenhaltungen:
- WO-A1-01/76385
- WO-A1-03/053157
- DE-A1-102011 050 905
- GB-A- 842 356
- US-A- 2 667 973

## Beschreibung

Die vorliegende Erfindung betrifft ein Verfahren zur Gewinnung von Wertprodukten, insbesondere Proteinen, aus einem nativen Stoffgemenge.

Dabei soll mit der vorliegenden Erfindung eine möglichst weitgehende Verarbeitung dieses Stoffgemenges zu Wertprodukten erfolgen.

Die DE 195 29 795 C2 offenbart ein Verfahren, welches die Gewinnung von Ölen, Fetten oder ermöglicht. Bei diesem Verfahren wird ein wässriger Brei in einer Zentrifuge in feste und flüssige Bestandteile aufgetrennt. Zu dem wässrigen Brei wird ein Anteil von 5-75 Gew%, bezogen auf den Flüssiganteil des Breis, eines organischen Lösungsmittels zugegeben. Die DE 195 29 795 C2 setzt dabei bei der Aufgabe an, aus dem wässrigen Brei eine blanke Ölphase, eine Wasserphase und eine von Öl befreite Feststoffphase zu isolieren. Dieses Verfahren hat sich grundsätzlich für die Gewinnung von Ölen, Wachsen und Fetten bewährt.

Bekannte Verfahren zur Proteinherstellung sind die Proteinisolatherstellung bei alkalischem pH-Wert oder die Proteinkonzentratherstellung bei saurem pH-Wert, welche vorzugsweise bei aus Hexan extrahierten Schrot angewandt werden, welche jedoch in Verbindung mit dem Verfahren der DE 195 29 795 C2 auf ein Protein-Lezithin-Gemisch ohne vorherigen energieintensiven Trocknungsschritt nicht anwendbar sind

In der Literatur sind weitere Veröffentlichungen zur Herstellung von Proteinprodukten aus entölten Rohstoffen angeführt, so etwa Kroll et al, "Rapssamenproteine - Struktur, Eigenschaften, Gewinnung und Modifizierung", Deutsche Lebensmittel-Rundschau, Heft 3, 2007, S. 109.

Diese Verfahren gehen i.d.R. vom entölten Rohmaterial aus. Typische Testölgehalte sind 1-4%. Sollte dies nicht so sein, werden Lösemittelextraktionen vorgenommen, um den Ölwert auf ein unvermeidbares Minimum zu reduzieren. Entölt müssen diese Rohstoffe deshalb sein, weil das Öl bei den gängigen Verfahren mehrfach stört. Es verbleibt immer in der wasserfreien Phase, ist also Teil der Trockensubstanz. Somit verbleibt es in dem Proteinkuchen oder Proteinkonzentrat, also als Verunreinigung des Proteines zurück.

Einige Verfahren nutzen auch Filtertechniken. Die Filter können sich mit dem Ölanteil zusetzen, der emulgiert vorliegt. Damit verbunden sind Proteinverluste im Proteinisolat.

So ist der gängige Ansatz zur Proteinkonzentratherstellung: Waschung der Schrote (stark entölt), wobei die löslichen Extraktionsstoffe abgereichert werden. Die Wertigkeit der entölten Zwischenprodukte hängt stark von der Konzentration an Begleitstoffen ab, wie Fasern, Zucker und sekundäre Pflanzenstoffen (Menner, M. u.a. "Fraktionierung pflanzlicher Rohstoffe zur simultanen Erzeugung von Lebensmitteln, technischen Rohstoffen und Energieträgern", Chemie Ingenieur Technik, Band 81, Ausgabe 11, Seiten 1743 - 1756, November 2009).

Zu diesen Begleitstoffen zählen auch Polyphenole wie Sinapin. Um diese Stoffe abzutrennen, werden große Verdünnungen gewählt, auch Proteine denaturiert (Temperatur, Alkohol), Zellulose wird enzymatisch abgebaut zu kurzkettigen Kohlenhydraten; diese Methoden werden gewählt, um die Stoffen besser extrahieren zu können.

Zurück bleiben Proteinkonzentrate, deren Proteinanteil dann erhöht wird, wenn im Vorfeld eine Schälung erfolgte, die den Schalen- bzw. den Zelluloseanteil vermindert.

Den Verfahren ist gemein, dass lösliche Proteine (Albumine, teilweise Globuline) mit den Polyphenolen, Kohlenhydraten und anderen gelösten Stoffen mit extrahiert werden.

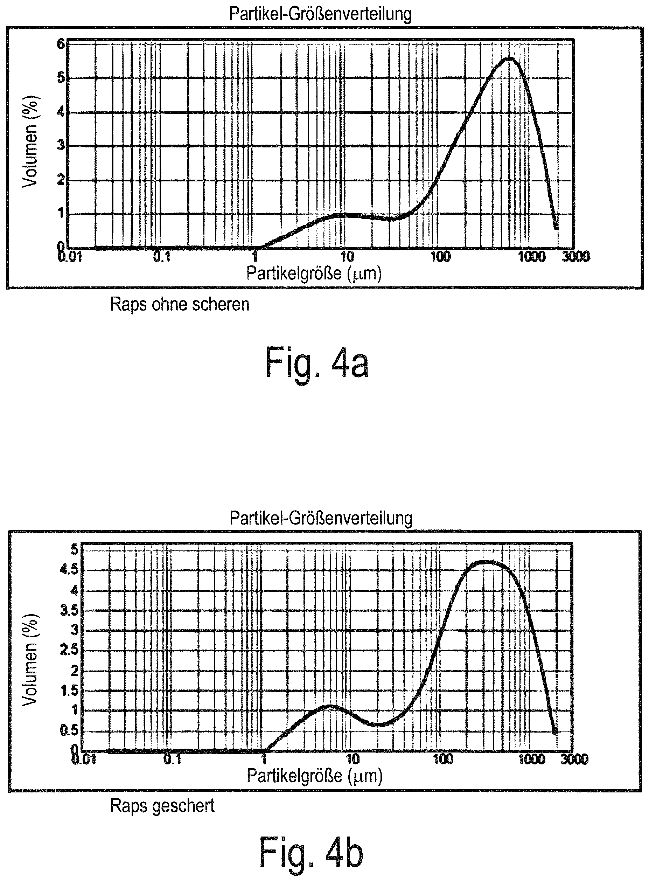

Andere Ansätze gehen von einer Feinstzerkleinerung aus, jedoch müssen dann auch hierbei die zellulosehaltigen Schalenbruchstücke vom Protein abgetrennt werden. Je kleiner diese sind, umso schwieriger wird die Klassierung bzw. die Stofftrennung im Allgemeinen. Die Proteinkonzentratphase bleibt verunreinigt. So führt eine mechanische Zerkleinerung des Schrotes/Kuchens oder ein intensives Scheren der Schrot- oder Kuchendispersion, ggf. noch verbunden mit einer enzymatischen Behandlung immer zu kleineren Zelluloseeinheiten bis hin zu kurzkettigen Kohlenhydraten Im Anhang (Fig. 4a, b) ist beispielhaft zu erkennen, dass gebrochener Kuchen ein Maximum in der granulometrische Verteilung bei etwa 600 µm hat und nur ein wenig ausgeprägtes relatives Maximum bei 8-10 µm.

Durch das Scheren wird der Volumenanteil des Globalen Maximums bei ca., 600 µm von ca. 5,5% auf ca. 4,5 % nachteilig reduziert und infolgedessen nimmt das relative Maximum der kleinen Partikeln bei ca. 8 µm auf über 1 % zu. Die Proteinphase wird dadurch grauer.

Diese kleinen Partikeln sind schwer vom Protein zu trennen. Es bleibt als Verfahren die Extraktion, in hohen Verdünnungen oder mehrstufig.

Aus der gattungsgemäßen DE 10 2011 050 905 A1 ist zudem ein Verfahren zur Gewinnung von Proteinen aus nativen Stoffgemengen bekannt, bei dem ein natives Stoffgemenge zunächst fein zerkleinert und gegebenenfalls durch Zugabe einer Flüssigkeit zu einem fließfähigen Brei verarbeitet wird. Das Verfahren weist ferner folgende Schritte auf: Einstellen des pH-Wertes des Breis in einen alkalischen Bereich; Zugeben mindestens eines wasserlöslichen organischen Lösemittels im Anschluss an das Einstellen des pH-Wertes des Breis; und Abtrennen einer Proteinphase aus dem Brei im Anschluss an das Zugeben des wasserlöslichen Lösemittels. Offenbart wird zu dem, aus dem Brei vor dieser Phasentrennung eine Feststoffphase mit einem Schalenanteil abzutrennen. Die Reihenfolge dieser Schritte einzuhalten, ist besonders vorteilhaft. Dabei erfolgt, anders als in der DE 195 29 795 C2, vor der Zugabe des wasserlöslichen organischen Lösemittels, ein Einstellen eines pH-Wertes des Breis in einen alkalischen Bereich. Dadurch wird die Löslichkeit der Proteine im wässrigen Medium erhöht, sie werden teilweise angelöst und liegen, sofern sie nicht vollständig aufgelöst sind, zumindest feinverteilt und voluminös in der Lösung vor und nicht in kompakter Form wie die übrigen Feststoffe. Eine vollständige Löslichkeit der Proteine ist durch das Vorliegen eines Protein-Lezithin-Gemisches gestört. In Anschluss an die Einstellung des pH-Wertes erfolgt die Zugabe des organischen wasserlöslichen Lösemittels, wodurch u.a. Öl, insbesondere die Triglyceride und unpolare Stoffe aus der angelösten Proteinsuspension verdrängt wird.

Das Verfahren der DE 10 2011 050 905 A1 ermöglicht es somit, Proteine mit hoher Reinheit zu gewinnen, da u.a. durch die Erhöhung der Löslichkeit der Proteine offenbar auch Bindungen beispielsweise zu Verunreinigungen aus Zellulose bzw. Schalen und dergleichen gelockert werden.

Gewöhnlich ist der erst Schritt ein Pressvorgang zur Entölung oder Teilentölung. Dabei bilden die Schalen ein Gerüst, um einen Presskuchen zu formen. Selbst bei geschälter Saat verbleibt ein notwendiges Minimum an Schalen zur Pressung.

Durch den Pressvorgang kommt es zum Verkleben der Proteine an den Schalen. Dabei gilt: je höher der Pressdruck oder die Temperatur sind, umso fester klebt das Kotyledon an den Schalen und umso schwieriger ist es, dies später zur Proteingewinnung von den Schalen abzutrennen. Dadurch sind Proteinverluste verursacht.

Vor diesem Hintergrund verbleibt dennoch als Aufgabe der Erfindung, die Gewinnung von Wertstoffen aus dem nativen Stoffgemenge weiter zu optimieren.

Die Erfindung löst diese Aufgabe durch die Merkmale des Anspruchs 1.

Weitere vorteilhafte Ausgestaltungen der Erfindung sind Gegenstand der Unteransprüche.

Nachfolgend soll eine vorteilhafte Verfahrensvariante der Erfindung detaillierter erläutert werden, wobei auch die Zeichnung genutzt wird. In dieser zeigen:
- Fig.1: einen Dekanter 1 mit einem Zulauf 2 darstellt, wobei die Schalen 3 von dem Restbrei 4 getrennt werden;
- Fig. 2: schematisch oben einen Behälter aus Schalen aus warm gepresstem Kuchen mit weißen Proteinanhaftungen und unten Schalen aus kalt gepresstem Kuchen ohne Proteinanhaftungen.
- Fig. 3: schematische Ansichten von Bechergläsern mit Proben.
- Fig. 4a, b: Diagramme, die den Einfluss eines Scherens auf die Zerkleinerung der Schalen veranschaulichen.

Das erfindungsgemäße Verfahren weist vorzugsweise folgende Schritte auf:

### Schritt A:

Als Ausgangsmaterial wird natives Stoffgemenge aus Saaten (Samen) mit harten, zerbrechbaren Schalen, insbesondere aus
- ganzen Leguminosensaaten/-früchten,
- ganzen Sonnenblumenkernen (Saaten/Früchte der *Helianthus annuus) oder Soyabohnen,* und/oder
- ganzen Saaten/Früchten von Kreuzblütengewächsen (Brassicaceae), insbesondere von Rapsfrüchten bereitgestellt.

Das Stoffgemenge im Sinne dieser Anmeldung kann aus den vollständigen, jedoch gebrochenen Saaten bestehen.

Alternativ kann das Stoffgemenge aber auch aus einem bereits entölten Produkt bestehen, insbesondere aus einem "Zwischenprodukt", d.h. aus einem Presskuchen, der nach einer "Vorstufe", z.B. dem Abpressen von Öl, insbesondere mit einer Presse (z.B. einer Schneckenpresse) als Rückstand der Ölgewinnung verbleibt.

Besonders bevorzugt wird als das Ausgangsmaterial "kurz zuvor gewonnenes Zwischenprodukt" verarbeitet, d.h. nach der Vorstufe dürfen nicht mehr als 31 Tage vergangen sein.

Die Saat kann frisch geerntet oder aber Tage, Wochen oder Monate alt sein, die Zwischenstufe (das Pressen) sollte kurz oder sogar unmittelbar vor der weiteren Verarbeitung stattfinden, damit sich nach der Ölgewinnung das Material - die Saat - nicht zu stark verändert hat.

Ganz bevorzugt wird als das Ausgangsmaterial "frisches Material" verarbeitet, d.h. nach eine Vorstufe bzw. Vorbearbeitung (Ölgewinnen) dürfen nicht mehr als 3 Tage vergangen sein, vorzugsweise sogar nur weniger als 48 Stunden oder 24 Stunden oder 12 h oder weniger als 1 h.

Mit Material aus einem Zeitraum kurz nach der Vorstufe werden gute oder und mit frischem Material in der Regel nochmals bessere Ergebnisse hinsichtlich der Ausbeute und der Reinheit der Wertprodukte erzielt.

Der Presskuchen kann einen Restölgehalt aufweisen, der auch bei 20% oder mehr liegen kann. Trotz derart hoher Restölgehalte ist die Gewinnung auch einer Proteinphase mit der Erfindung auf einfache Weise realisierbar.

### Schritt B:

Sofern es noch nicht zerkleinert vorliegt: Zerkleinern des Stoffgemenges aus Schritt a) zum Aufbrechen der Schalen. Sofern ein Presskuchen verwendet wird, wird dieser aufgebrochen, idealerweise unmittelbar nach dem Pressen, noch warm. Derart wird ein zerkleinertes Material - eine Art Granulat - aus dem Presskuchen erzeugt. Das zuvor durch einen Pressvorgang (teil-) entölte Stoffgemenge wird in der Regel nur zerkleinert, beispielsweise zermahlen oder es werden jedenfalls die Schalen aufgebrochen.

### Schritt C:

Das bereitgestellte und zerkleinerte Stoffgemenge aus Schritt A) oder B) wird mit Wasser dispergiert. Auf einen Teil "zerkleinertes Produkt" werden vorzugswiese bis zu maximal 8, vorzugsweise bis zu maximal 5 Teile Wasser zugegeben. Sodann werden Wasser und zerkleinertes Produkt gerührt, so dass sich ein fließfähiger Brei bzw. eine Dispersion ergibt. Das Rühren erfolgt vorzugsweise für mehr als 30 min, insbesondere für mehr als 1 h.

### Schritt D)

Als nächstes erfolgt ein Einstellen des pH-Wertes des Breis (I) aus Schritt c) in einen alkalischen Bereich; Vorzugsweise wird der pH-Wert des Breis bzw. der Dispersion mit Lauge auf pH 10 bis 11 eingestellt. Dabei wird (vorsichtig) das Rühren fortgesetzt. Die Verrührzeit beträgt vorzugsweise mehr als 30 min, vorzugsweise liegt sie bei 1 h oder darüber.

### Schritt E)

In diesem weiteren Schritt erfolgt ein Zugeben mindestens eines wasserlöslichen organischen Lösemittels im Anschluss an das Einstellen des pH-Wertes des Breis im Schritt D. Vorzugsweise wird die Dispersion, deren pH-Wert in den alkalischen Bereich eingestellt worden ist, mit dem Alkohol EtOH (vorzugsweise 30-60%ig) auf eine Alkoholkonzentration von 20-15% oder weniger, insbesondere 12% EtOH Konzentration gebracht. Entsprechend der Wassermenge des verwendeten Alkohols kann die Wassermenge in Schritt C um das im Alkohol, insbesondere im 30-60%igen EtOH enthaltene Wasser, reduziert werden. Damit lösen sich die Schalen vom Endosperm (Kotyledon) mit dem Restöl und können abgetrennt werden, insbesondere zentrifugal.

### Schritt F)

Im Schritt F) erfolgt daher ein Abtrennen einer Feststoffphase, welche den überwiegenden Anteil der Schalen umfasst, vorzugsweise in einer Zentrifuge im Zentrifugalfeld aus dem Brei bzw. es erfolgt ein Klären des Breis von Schalen-Feststoffanteilen insbesondere in einem Dekanter.

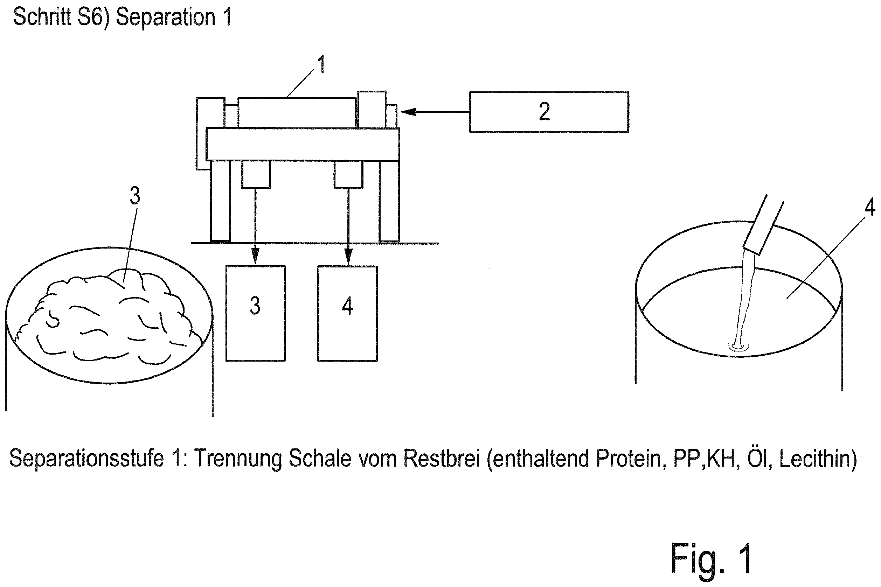

Diesen Schritt veranschaulicht Fig.1, welche einen Dekanter 1 mit einem Zulauf 2 darstellt, wobei die Schalen 3 von dem Restbrei 4 getrennt werden.

Die leichtere Phase einer zentrifugalen Phasentrennung wird nachfolgend auch gelegentlich als Oberlauf bezeichnet und die Feststoffphase als schwere Phase. Eine Mittelphase läge entsprechend bzgl. ihrer Dichte dazwischen.

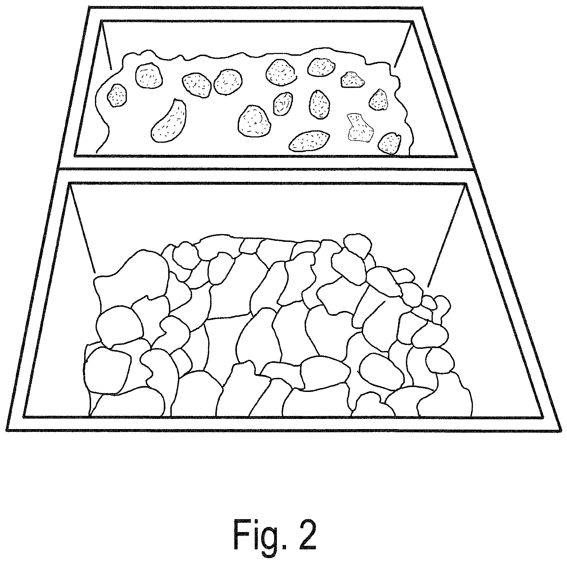

Fig. 2 zeigt rein schematisch oben einen Behälter aus Schalen aus warm gepresstem Kuchen mit weißen Proteinanhaftungen und unten Schalen aus kalt gepresstem Kuchen ohne Proteinanhaftungen.

### Schritt G)

Der jedenfalls weitestgehend schalenfreie Brei aus Schritt F) wird nunmehr weiterverarbeitet. Vorzugsweise erfolgt dabei eine Ausfällung des gelösten - Proteinanteiles aus dem schalenfreien Brei, der zusammen mit dem un- oder angelösten Protein-Teil eine Fraktion bildet, den Quark. Der pH-Wert wird dabei wieder weiter in den sauren Bereich verschoben, insbesondere in den pH-Bereich von pH = 4,5 bis pH = 7.

### Schritt H)

Sodann wird der schalenfreie Brei, dessen pH-Wert wieder ins Saure verschoben worden ist, - vorzugweise in einer Zentrifuge, insbesondere in wenigstens einem Dekanter oder in einem Separator - in einem oder zwei Schritten in Wertstoffphasen getrennt, von denen eine Phase eine Proteinkonzentratphase ist.

Die Proteinkonzentratphase wird unter Verwendung eines Mahltrockners zu einem Pulver getrocknet. Besonders bevorzugt erfolgt eine Trennung in folgende zwei oder drei Phasen:
- ölhaltige Phase
- wässrige Phase (Polyphenol, Kohlenhydrat und Sinapinsäurehaltig)
- Proteinkonzentratphase (nachfolgenden auch "Proteinquark" genannt) oder
- wässrige Phase mit Albumingehalt und Restölgehalt; und
- Proteinkonzentratphase (Proteinquark);

Die Zwei-Phasentrennung wird dann geführt, wenn das Rohmaterial relativ stark entölt ist und/oder im Feststoff gebunden vorliegt oder wenn kein intensiver Scherungseinfluss für die Flüssigkeitsphase in Schritt 1 ausgeführt worden ist. Die Zugabe von Wasser oder Alkohol oder Lauge oder dgl. kann auch in Teilschritten erfolgen. Das Öl als leichtere Phase enthält Triglyceride und ist einer der gewinnbaren Wertstoffe. Vorzugsweise liegt die Temperatur während sämtlicher Verfahrensschritte unter 60°C, insbesondere unter 50°C, vorzugsweise, zwischen 40 °C und 50°C, wodurch sich besonders wertvolle Produkte gewinnen lassen.

Die Denaturierung der Proteine ist ein temperatur- und zeitabhängiger Prozess. Hinzu kommt die Bedingung im alkoholischen Milieu. Die Proteindenaturierung geht umso schneller, je höher die Temperatur ist. In wässriger Umgebung ist aber auch bei Wärmeinwirkungen von 45-50 °C keine irreversible Proteindenaturierung zu erwarten. Das ändert sich aber mit der Alkoholkonzentration. Schon bei Umgebungstemperatur ist bei hochkonzentriertem Alkohol eine Proteinausfällung zu beobachten. Je geringer nun die Alkoholkonzentration ist, umso höher muss die Temperatur sein, um die Proteine zu denaturieren. Oder umgekehrt: je wässriger die Alkoholkonzentration ist, umso höher darf die Arbeitstemperatur sein, ohne dass die Proteine irreversibel geschädigt werden.

Man wird also (für reines Wasser) eine möglichst hohe, d.h. möglichst an 60°C heranreichende Temperatur wählen, um möglichst viele Stoffe in Lösung zu bringen, wie Proteine, Lecithine, Glycolipide etc.. Damit können die Zellulose, das Lignin und Salze wie Ca-Phytate als unlösliche Bestandteile der oder mit der Schalenfraktion abgetrennt werden. Es ist jedoch darauf zu achten, dass die Temperatur entsprechend den Prozessparametern Zeit und Alkoholkonzentration (ggf. Druck) hinreichend niedrig bleibt.

Die gefällten Proteine liegen als Proteinquark vor (schwere Phase). Sie bilden einen weiteren der gewinnbaren Wertstoffe. Diese Phase kann gut zu Pulver getrocknet werden.

Insgesamt wird eine auch optisch ansprechende und daher gut weiter verwertbare Proteinkonzentratphase gewonnen, die in einer Farbeinstufungsskala RAL den Werten RAL 1015 (Hellelfenbein) oder RAL 1013 (Perlweiß) zuzuordnen ist. Als RAL-Farbe werden normierte Farben bezeichnet (RAL GmbH, Tochter des RAL-Institutes) Jeder Farbe ist eine vierstellige Farbnummer zugeordnet. Theoretisch kann für das Verfahren jeder Presskuchen verwendet werden.

Die vorteilhafte Temperaturangabe zu den Verfahrensschritten A bis H bezieht sich nicht auf die Presstemperatur beim Erzeugen des Presskuchens bei der Ölerzeugung. Je höher die Temperatur bei den vorangegangen Prozessschritten war, umso brauner wird die Proteinphase bzw. Quarkfraktion. Dies liegt einerseits an der Maillard-Reaktion von Zuckern mit Proteinen, andererseits and der Phenoloxidation. Gegenüber der DE 10 2011 050 905 A1 wird insbesondere durch die Verwendung optimiert ausgewählten Ausgangsmaterials (vorzugsweise kalt gepresster RapsPresskuchen, vorzugsweise sehr frisch) ein besonders ansprechendes, besonders gut weiterverwertbares Produkt gewonnen.

Besonders vorteilhaft ist die Verwendung kalt gepressten Materials (insbesondere eines kalt gepressten Rapspresskuchens (Temperatur beim Pressen vorteilhaft kleiner als 70 °C, besonders vorzugsweise sogar kleiner als 60 °C) als Ausgangsmaterial bzw. als das bereitgestellte Stoffgemenge. Warm gepresstes Material wird beim Pressen deutlichen höheren Temperaturen (bis über 100°C) ausgesetzt. Durch die Verwendung kalt gepressten Materials als Ausgangsmaterial für das erfindungsgemäße Verfahren kann eine Proteinphase bzw. "Protein- bzw. Quarkphase" mit deutlich besseren Eigenschaften (insbesondere hinsichtlich der Farbe deutlich heller und daher besser verarbeitbar) und mit deutlich besserer Ausbeute gewonnen werden als bei der Verwendung warm bzw. heiß gepressten Ausgangsmaterials. Dies wurde im Stand der Technik bisher nicht erkannt. Denn gängige Rapspressverfahren zielen auf eine hohe Ölausbeute, weshalb beim Pressen gern höhere Temperaturen verwendet werden. Als Nebeneffekt ist festzustellen, dass Sinapin (ein Polyphenol) abgebaut wird, was an sich vorteilhaft für die Proteinfraktion wäre. Beim erfindungsgemäßen Verfahren stellt der originale, also nicht reduzierte, Sinapingehalt im kalt gepressten Kuchen aber dennoch kein Problem für das Endprodukt dar, da die polyphenolischen Verbindungen im Wesentlichen nicht in der Quarkphase zu finden sind, da sie in die Wasserphase übergehen.

So ist die Quarkphase, die nach dem erfindungsgemäßen Verfahren aus einem zuvor mit Hexan zusätzlich entöltem Presskuchen gewonnen wurde eher dem RAL-Ton 1024 ockergelb oder 1014 Elfenbein zuzuordnen. Die Verarbeitung erfolgt vorzugsweise unter Umgebungsdruck.

Auch in der Wasserphase sind noch wertvolle Inhaltsstoffe enthalten, insbesondere ist sie relativ stark albuminhaltig. Sinnvoll und vorteilhaft ist insofern eine Filtration der Wasserphase aus S8 zur Albumin-Aufkonzentrierung, um derart die Albuminphase als weiteren Wertstoff zu gewinnen.

Eine besonders vorteilhafte Verfahrensvariante sei anhand des folgenden Beispiels erläutert. Dabei werden mehrere Schritte "S" durchlaufen:
S1. Ausgangsmaterial ist bei diesem Beispiel gepresster Rapskuchen oder (auch Sonnenblumenschrot oder Soyamehl), idealerweise schonend und kalt gepresst, mit typischen Restölgehalten von 20%; auch höher stellt kein Problem dar).
S2. Der Kuchen wird aufgebrochen, idealerweise unmittelbar nach dem Pressen, noch warm.
S3. Das Kuchengranulat wird mit Wasser dispergiert (1 Teil Kuchen und max. 6 Teile Wasser) und vorsichtig zu rühren (1 h).
S4. Diese Dispersion ist mit Lauge auf pH 10 bis 11 einzustellen und vorsichtig zu rühren, üblicherweise 1 h.
S5. Die Dispersion aus 4 ist mit EtOH (vorzugsweise 30-60%ig) auf 12% EtOH Konzentration zu bringen, somit wird die Wassermenge in Punkt 3. um das in diesem 30-60%igen EtOH enthaltene Wasser reduziert.
S6. Damit lösen sich die Schalen vom Endosperm (Kotyledon) mit dem Restöl und können zentrifugal abgetrennt werden.
S7. Ausfällung vom Protein durch Ansäuern auf vorzugsweise pH = 4,5 bis 7,2 aus dem Oberlauf (Oberlauf: Leichte Phase der Separation aus Schritt S6 mit einem pH-Wert von vorzugsweise 9,7 bis 10,5) zur Trennung: Öl - Wässrige Phase - Proteinkonzentratphase (Proteinquark) oder Trennung in Öl/Wasserphase und Proteinkonzentratphase; Unterstützt werden kann dieser Schritt durch einer intensive Scherung, um die Ölfreisetzung zu erleichtern.
S8. Abtrennung der gefällten Proteine als Quark (schwere Phase (in der Regel Feststoffphase bzw. hier Quarkphase)) und ggf. Triglyceride (als leichtes Öl) aus dem Oberlauf (leichte Phase), insbesondere zentrifugal.
S9. Filtration der Wasserphase aus S8 zur Albumin-Aufkonzentrierung.

Als optionaler Schritt 10 könnte hier die wiederholte Waschung des Quarkes mit leichten EtOH-Wasser genannt werden = Reinheitserhöhung

Als besonders vorteilhaft zu erwähnen ist die Nasstrennung der Schalen von den gelösten und gequollenen Proteinen bei parallel stattfindender Verdrängungsextraktion der Triglyceride (Ölphase) aus Öl- oder restölhaltigem Presskuchen oder Leguminosen Mehl und parallel verlaufender Polyphenolextraktion

Vorteile des erfindungsgemäßen Verfahrens sind.
1. Geringe Verdünnungen: damit geringe Volumenströme im Prozess,
2. Höhere Polyphenolkonzentrationen während der Extraktion in der wässrigen Phase (Verfahrensschritte 2 bis 5),
3. Native Proteine im Endprodukt, da der Prozess bei max. 50-55°C oder weniger umgesetzt wird,
4. Hohe Proteinausbeuten mit bis zu 45% (bis zu 70%) in der "Quarkphase" zzgl. ca. 22-24% in der Albuminphase,
5. Höherwertiges Endprodukt, weil Schalen- und Polyphenol, Kohlenhydrat, Phytinsäure oder Phytate, Legnin und Zellulose vollständig entfernt oder abgereichert sind, enthält "natives" Protein dessen quellfähiger Anteil bleibt quellfähig, dessen wässrig lösbarere Anteil bleibt wasserlöslich, nahezu triglyceridfrei (Öl-), geringe Restölwerte (hauptsächlich Polarlipide),
6. Ungünstiges Milieu für Mikroorganismenwachstum durch die leichte Alkoholkonzentration vereinfacht die Prozesshygiene, und
7. Der Alkohol kann verdünnt im Kreislauf gefahren werden.

### Zu den Schritten S1- S2

Anstelle der Extraktion unerwünschter Stoffe aus dem stark entölten, feinst zerkleinerten Ausgangsmaterial Rapsschrot oder Rapskuchen -wie bei den gängigen Verfahren üblich-, erfolgt hier zunächst eine Abtrennung der Schalen im Nassen. Dies wird in einem mehrstufigen Prozess dadurch gelöst, dass zunächst der Kuchen aufgebrochen wird, ohne die Kernbruchstücke noch weiter zu zerkleinern.

Insbesondere kommt es darauf an, die Schalen möglichst groß zu belassen. Vorzugsweise sollten sie einen mittleren Durchmesser von 0,5 mm oder mehr aufweisen. Öltröpfchen brauchen nicht größer sein, wichtig sind nicht einzelne Moleküle oder kleine Molekülverbände, sonder "Partikel".

Dann wird Wasser zugegeben und im Alkalischen wird vorsichtig gerührt. Damit wird der wasserlösliche Teil der Proteine gelöst, ein anderer Teil quillt. Die Zugabe von wässrigem Alkohol verdrängt das freie Triglycerid als spezifisch leichte Phase aus der Dispersion. Die Lecithine, insbesondere Phosphatidylcholine, sind bei geringen Alkoholkonzentrationen löslich (siehe EP 1272048 B1 und zugehörige Patentfamilie).

In dieser Kombination Lauge-wässriger Alkohol sind die zwei oder drei Phasen
1) Schwer = Schalen und 2) Leicht = Protein-Lecithin-Polyphenol- Kohlenhydrat zusammen mit ölhaltigem Schaum; oder
1) Schwer = Schalen, 2) Mitte = Protein-Lecithin-Polyphenol- Kohlenhydrat 3) Leicht = Triglycerid, vorteilhaft trennbar, so im Versuch im Becherglas oder im industriellen Maßstab vorzugsweise zentrifugal.

Je besser die Abtrennung der Schalen gelingt, umso geringer sind die Proteinverluste und umso reiner ist das Endprodukt. Selbst die durch Wasserzugabe auf das bis zu 7 fache gequollene Schale ist schwerer als die Proteine in der alkoholischwässrigen Dispersion. Dies ist essentiell für eine Trennung durch Gravitation. Erschwert wird die Trennung aber durch ein festes Kleben der proteinhaltigen Aleuron Körper(Wabenschicht) an den Schalen. Diese Zellen sind dickwandig. Da die Zellmembran fast aller Zellen Lecithine enthält (neben Proteinen und anderen), kann nun durch geeignete Maßnahmen das Kleben durch ein "In-Lösung-Bringen" der Lecithine minimiert werden.

Konkret wird dies dadurch erreicht, dass die wässrige Phase eine Alkoholkonzentration von 5-40 % aufweist. (siehe Schritte S2-S4), idealerweise 12% bis 20%.

Entscheidend dafür ist bereits die Qualität des Ausgangsmaterials. Gewöhnlich ist bei kalt gepresstem Kuchen der Restölgehalt höher. Dies stört bei dem hier dargestellten Verfahren nicht. Im Gegenteil: Äußerst hilfreich ist die schönende Pressung, je moderater die Presstemperatur und desto geringer ist der Pressdruck, umso leichter ist eine nachfolgende Trennung von Schalen und Kotyledon (Keimblätter, das Kerninnere).

Das Verfahren ist auch mit "gewöhnlichem" d.h. heiß gepresstem Presskuchen anwendbar. Nur werden hierbei die Ausbeuten an Proteinen entsprechend geringer.

### Zu den Schritten S2 bis S4) Dispersionsherstellung

Die Dispersionsherstellung mit Wasser- Lauge und Alkohol hat zwei Ziele: einerseits das Lösen von der Schale, andererseits das Extrahieren phenolischer Verbindungen wie Sinapin aus dem Rohmaterial. Dabei ist die Benetzung mit Fluid wichtig. Jedoch erzeugte ein Scheren bei der der Dispersionsformulierung in den Schritten 2-5 Kleinstpartikeln, die zur Verunreinigungen in den separierten Phasen führten. Ohne eine Scherkopfmischer-Anwendung bzw., ohne die Verwendung einer Zahnscheibenmischers für die Schritte 2-5 betrug der Proteingehalt in der Proteinphase (nach Schritt 7 und Trocknung) : >60% bei frischem Material.

Unter Verwendung eines Scherkopfmischers bzw. mit Anwendung eines Frisam-Schermischers betrug der Proteingehalt in der Proteinphase (nach Schritt 7 und Trocknung): ca. 50% bei altem Material

Ein anderer Test wurde mit heiß gepresstem Expeller-Kuchen durchgeführt. Die Menge an abtrennbaren Schalen in der ersten Stufe wird durch das Scheren von 20% auf 16% vermindert, gleichzeitig erhöht sich die Menge an ausfällbarem Protein aus der Klarphase von 38% auf 42%. Die Reinheit bleibt mit 39-40% relative konstant und niedrig.

### T = 50°C, 30 min Reaktionszeit (Zu Schritt S5) Alkoholmilieu

Neben der Lösung der Lecithine im Wässrige-Alkoholischen hat zur verbesserten Abtrennung von Schalen einerseits und Triglyceriden anderseits hat die Abtrennung im leicht Alkoholischen zusätzlich den Vorteil, dass ein Wachstum von Mikroorganismen im Prozess erschwert wird. Dies ist ein deutlicher Vorteil gegenüber den rein wässrigen Verfahren und erleichtert die CIP-Reinigung

### Zu Schritt Separation 1

In Fig.1 bedeutet "Kalt = kalt gepresster Kuchen; warm = warm gepresster Kuchen; heiß = konditionierter und heiß gepresster Kuchen" Beispiel:
B1) Kalt gepresster Kuchen: 17% Schwere Phase als Schalenanteil vom Zulauf mit 20 % der Kuchen-Proteine und 83% Oberlauf als Protein-Polyphenol-Öl-Phosphatid-Phase mit 80 % der Kuchenproteine
B2) warm gepresster Kuchen: 26% Schwere Phase als Schalenanteil vom Zulauf mit 30 % der Kuchen-Proteine und 74% Oberlauf als Protein-Polyphenol-Öl-Phosphatid-Phase mit 70 % der Kuchenproteine
B3) heiß gepresster Kuchen: 30% Schwere Phase als Schalenanteil vom Zulauf mit 50 % der Kuchen-Proteine und 70% Oberlauf als Protein-Polyphenol-Öl-Phosphatid-Phase mit 50 % der Kuchenproteine.

### Zu Schritt S7) Proteinfällung

Aus dem Oberlauf (Oberlauf = leichte Phase) der Separation im Schritt S6 werden die Proteine durch ph-Verschiebung auf den Bereich von 4,5 bis ca. 7 ausgefällt. Die wasserunlöslichen Proteine (jedoch quellbaren) bilden zusammen mit den ausgefallenen Globulinen die Proteinfraktion des "Proteinquarks". Die Flüssigkeit in dieser Fraktion hat dieselbe Zusammensetzung wie die Flüssigkeit der Mittelphase (Oberlauf ohne Triglyceride). Da aber die Quarkphase nur 10-30 % des Zulaufes ausmacht, (mit 15-25% Trockensubstanz), sind in der Quarkphase mengenmäßig auch wesentlich weniger Polyphenole zu finden als in der Mittelphase, wenngleich die Konzentration der Polyphenole bezogen auf das Wasser dieselbe ist.

Damit ist eine Proteinphase aus wasserunlöslichen jedoch gequollenen Proteinen mit Globulinen verfügbar, die Polyphenol abgereichert ist. Diese Kombination aus alkalisch-ethanolischem Milieu in den Schritten S2-S5 . gefolgt von einem saueralkoholischen Milieu zur Proteinfällung stellen sehr gute Bedingungen für eine Polyphenol-Extraktion dar. Überraschenderweise hat sich für Raps (Sinapin und Derivate) hier die Beobachtung für andere Polyphenole (Tyrosol und Derivate u.a,) aus anderen Bereichen wie der Verarbeitung von Oliven bestätigt, obwohl deutlich mehr reaktionsaktive Stoffe wie Proteine und Zucker in der Suspension enthalten sind.

Damit werden Verdünnungen wie in der Literatur beschrieben hinfällig, um auf gleichwertige Polyphenol Extraktionsraten des Wässrigen zu kommen (so etwa wiederum Kroll et al, "Rapssamenproteine - Struktur, Eigenschaften, Gewinnung und Modifizierung", Deutsche Lebensmittel-Rundschau, Heft 3, 2007, S. 109.

Da das reine Triglycerid aus der Flüssigkeit als leichte Phase verdrängt wird, kann der Restölgehalt im Protein-Endprodukt auf unter 15%, auch unter 13% bezogen auf Trockensubstanz gesenkt werden.

Da die Temperaturen während des gesamten Prozesses < 50 °C betragen, kann auch von einem nativen Endprodukt gesprochen werden.

Vorteilhaft ist ein Scheren des weiter zu verarbeitenden Breis vor der Phasentrennung des Schrittes H (vor der Ölabtrennung) und nach Schritt F) oder G) des Anspruchs 1 zur Verbesserung der Verdrängungsextraktion. Dieses Scheren kann mit einer Schervorrichtung wie einem Homogenisator oder einem Intensivmischer durchgeführt werden, um derart noch mehr Öl zu gewinnen.

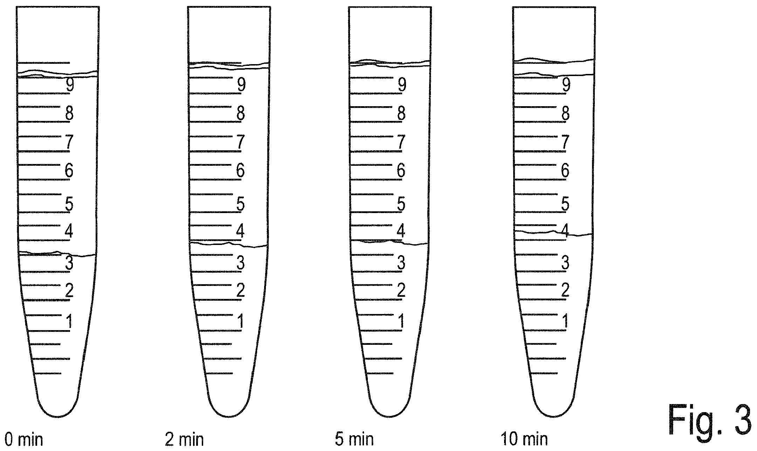

Fig. 3 zeigt Proben, bei welchen verschieden lang ein Scheren durchgeführt wurde (0 min, 2min, 5 min, 10 min). Es ist zu erkennen, dass mehr Öl im rechten Bild (obere Ringe im Probenglas) freigesetzt wird. Durch den Scherprozess werden lokal hohe Drücke erzeugt.

Das Scheren mit einer Schervorrichtung kann im kontinuierlichen Prozess durchgeführt wird. Insgesamt wird vorzugsweise ein kontinuierlicher Prozess realisiert.

S8: Separation der Proteine als Quark mittels Dekanter oder Separator

Zur Erhöhung der Reinheit kann der Proteinquark gewaschen werden. Der Quark kann anschließend zu einem Pulver getrocknet werden.

S9: Anschließend kann vorteilhaft eine Albumingewinnung erfolgen.

Kurzgefasst wird auch geschaffen ein vorteilhaftes Verfahren zur Gewinnung von Proteinen aus nativen Stoffgemengen, mit folgenden Schritten: A) Bereitstellen eines nativen Stoffgemenge aus Saaten mit harten, zerbrechbaren Schalen, B) Zerkleinern des Stoffgemenges, um jedenfalls die Schalen aufzubrechen, ohne sie zu fein zu dispergieren (vorzugsweise sollen die Größe der zerkleinerten Schalenanteile in einer granulometrischen Verteilung nach Art der Fig. 4 zwischen 100 und 2000 µm liegen. insbesondere mit einem Maximum zwischen 300 µm und 900 µm, insbesondere bei ca. 600 µm, jeweils bei einer relativen Häufigkeit von mehr als 5%); C) Dispergieren des zerkleinerten Stoffgemenges aus Schritt A) oder B) mit Wasser, D) Einstellen des pH-Wertes des Breis (I) aus Schritt C) in einen alkalischen Bereich pH > 9, 5; E) Zugeben des wasserlöslichen organischen Lösemittels Alkohol zu dem Brei aus Schritt C) im Anschluss an das Einstellen des pH-Wertes des Breis im Schritt D; F) Abtrennen einer Feststoffphase, welche den überwiegenden Anteil der Schalen aufweist, vorzugsweise in einer Zentrifuge im Zentrifugalfeld; G) Verschieben des pH-Wertes des von Schalen befreiten Breis aus Schritt F) in den pH-Bereich von pH = 4,5 bis pH = 7,2, und H) Trennen des schalenfreien Breis, dessen pH-Wert wieder ins Saure verschoben worden ist, - vorzugweise in einer Zentrifuge, insbesondere in wenigstens einem Dekanter- in einem oder zwei Schritten in folgende drei Wertstoffphasen: ölhaltige Phase mit Triglyceringehalt; wässrige Phase mit Albumingehalt und Proteinkonzentratphase (Proteinquark).

Es ist weiter vorteilhaft, wenn die Quarkphase getrocknet wird. Hier ist es vorteilhaft, noch vorhandenen Alkohol aus dem Quark abzuverdampfen, vorzugsweise unter Vakuum, um die Temperatur niedrig zu halten und dann den alkoholfreien, wässrigen Quark zu einem Pulver zu trocknen. Hierzu wird erfindungsgemäßein Mahltrockner genutzt. Derart wird ein lagerstabiles, leicht handhabbares sowie transportierbares Produkt geschaffen.

Die positiven Eigenschaften können anhand einer im Versuch gewonnenen Proteinphase veranschaulicht werden:
Versuch 1 (zu den Schritten A- -F): Versuchsansatz: 95 kg Stadtwasser+ 23 kg Rapssaat (Warmpressung) in Rührvorlage abgefüllt und auf 40 °C erhitzt (Schritte A und C). Anschließend wird dieses Produkt-/Wassergemisch mittels einer Monopumpe und eines Fristam-Mischers bei 1000 l/h ca. 8 min im Kreislauf gefahren (pH = 6,2). Sodann erfolgt eine Zugabe von 4,1 l 10 %iger NAOH auf pH = 10 (Schritt D). Anschließend wird 15 min. ohne Fristam Mischer bei 1000 l/h im Kreislauf gefahren und gerührt. Dann erfolgt eine Zugabe von 14,2 kg Ethanol (Schritt E in einem oder mehreren Teilschritten) mittels Schlauchpumpe direkt in den Kreislauf der Monopumpe. Verweilzeit: 10 min. Nach ca. 50 min Verweilzeit wird nochmals 2 kg Ethanol in 11 kg Wasser vermischt und in einen Rührwerksbehälter gegeben. 10 min Verweilzeit. Diese Suspension wird zentrifugal getrennt (Schritt F) Dabei beträgt die Ausbeute: 96,5 kg Klarphase, 34 kg Feststoffe. Die Schalen lassen sich somit leicht abtrennen.

Versuch 2: (A - F) Versuchsansatz: 116 kg Stadtwasser+ 26 kg Rapssaat (Kaltpressung) in Rührvorlage abgefüllt und auf 40 °C erhitzt. Anschließend mittels Monopumpe und Fristam-Mischer bei 1000 l/h ca. 8 min im Kreislauf gefahren (pH = 5,8). Zugabe von 4,5 I 10 % iger NAOH auf pH = 10. Anschließend 15 min. ohne Fristam Mischer bei 1000 l/h im Kreislauf gefahren .Zugabe von 17,2 kg Ethanol mittels Schlauchpumpe direkt in den Kreislauf der Monopumpe. 10 min Verweilzeit. Danach wird separiert, um die Schalenfraktion abzutrennen. Die Ausbeute beträgt Ausbeute: 129,6 kg Klarphase, 26,5 kg Feststoffe. Die Schalen lassen sich somit leichter abtrennen.

Versuch 3: (Schritte G und H zu Versuch 1): 96,5 kg Klarphase vom Versuch I (pH-Anfangswert: 9,6) wurden mittels 0,8 I 25 % iger Salzsäure auf pH 5 verschoben, hier vorteilhaft bei 45°C) (Schritt G). Dieser Brei kann sodann zentrifugiert werden, wobei eine Proteinphase als schwere Phase bzw. Feststoffphase gewonnen wird (Schritt H). Ausbeute Klarphase: 64,3 kg, . Ausbeute Feststoffphase (Quarkartig) 9 kg

Versuch 4 (Schritte G und H zu Versuch 2): 129,6 kg Klarphase vom Versuch II (pH-Anfangswert: 9,5) wurden mittels 1,21 25 % iger Salzsäure auf pH 5 verschoben bei 45°C (Schritt G). Dieser Brei kann sodann zentrifugiert werden, wobei eine Proteinphase als schwere Phase bzw. Feststoffphase gewonnen wird (Schritt H). Ausbeute: 83 kg Klarphase, 29,5 kg Feststoff )Proteinphase). Hier ist die Ausbeute an Feststoffphase besonders groß.

Typischerweise weist ein nach Art der vorstehenden Versuche gewonnenen und sodann getrockneten Quark gewonnene Pulver Trockensubstanzgehalte von 5-9 % auf; in einem Muster hergestellt aus gewöhnlichem Presskuchen 5,35%. Die Proteingehalt beträgt ca. 60% Das Wasserbindevermögen wurde mit 1,8 +/-0,2 ml H₂O auf 1 g Trockensubstanz des Proteinpulver ermittelt, das Ölbindevermögen mit 0,49 bis 0,63 g Öl/ g Trockensubstanz sowie die Emulgierbarkeit mit 700 bis 780 ml ÖL / g Trockensubstanz des Proteinpulvers. Typische Werte für den Proteinlöslichkeitswert NSI sind 9 bis 16%. Die besten Ergebnisse werden mit dem kalt gepressten Presskuchen erzielt.

In weiteren Versuchen zeigte sich überraschend, dass auch die Rührtechnik bei dem Verfahren von Bedeutung ist, was sich insbesondere auf das Rühren des Schrittes C) (und ggf. D) und E)) des Anspruchs 1 bezieht.

So wurde kalt gepresster Rapspresskuchen in der im Anspruch 1 beschriebenen Verfahrensweise verarbeitet. Dabei wurde im Schritt C) einmal mit einem Flügel-Rührer gerührt und einmal mit einem Propeller-Rührer.

Der Flügel-Rührer sollte so betrieben werden, dass er möglichst wenige Scherkräfte beim Rühren erzeugt sondern eine im Wesentlichen gleichmäßig laminare Strömung.

Bei dem Propeller-Rührer im Sinne dieser Anmeldung sind Rührelemente auch außerhalb der Drehachse verbunden, so durch eine Scheibe oder einen Ring oder in Ihrer Nähe sind Elemente wie eine offene Glocke über den Propellerelementen vorhanden. Sie erzeugen daher beim Rühren eine relativ turbulente Strömung und üben höhere Scherkräfte auf das Produkt aus.

Flügel-Rührer sind also solche, die im Wesentlichen eine laminare Strömung beim Rühren erzeugen, die relativ lange Flügel aufweisen und die bei geringer Drehzahl betrieben werden. Ein Ring oder eine Scheibe oder dgl. am Außenumfang der Flügel oder in deren Nähe (nach Art eines offenen Käfigs oder einer offenen Glocke um die Flügel) ist in der Regel nicht vorhanden. Typische Drehzahlen sind 0-100, auch bis 150 U/min. Im Gegensatz dazu erzeugt der Propeller-Rührer, dessen Flügeldurchmesser eher klein und dessen Rotor-Rotationsdrehzahl mit bis zu ca. 700-800 U/min eher hoch sind, hohe Turbulenzen, weshalb die Suspension stärker geschert wird.

Bei den weiteren Versuchen erfolgte nach den Schritten D) und E) - vorzugsweise unter weiterem Rühren mit dem Flügel-Rührer oder dem Propeller-Rührer - das Abtrennen der schalenhaltigen Feststoffphase gemäß Schritt F).

Die Flüssigphase nach der Schalenabtrennung aus einer Suspension aus Rapspresskuchen, der 13,4 % Öl, 31,4 % Protein und 55,2% Sonstiges enthielt (wie Zellulose, Phytinsäure, Polyphenole, Saccharide etc.), war bei der Verwendung des Flügel-Rührers zum Rühren in Schritt C) und ggf. D) und E) deutlich proteinreicher als bei Verwendung eines Propeller-Rührers. Ca. 75% der Proteine des Kuchens wurden in diesem Oberlauf gefunden, dessen Trockenmasse sich aus 52,3 % Protein, 13,0 % Öl und ca. 34% Sonstigem zusammensetzt. Im Gegensatz wurden nur 62,5% der Proteine des Kuchens in dem vergleichbaren Oberlauf gefunden, wenn ein Propeller-Rührer eingesetzt wurde. Für diesen Fall hatte die Oberlauf-Trockenmasse nur ca. 37% Protein, ca., 14,7 % Öl sowie 48,0 sonstige Inhaltsstoffe.

Auch optisch zeigten sich überraschend deutliche Unterschiede. Die Schalenfraktion der Schleuderprobe aus der Suspension unter Verwendung des Flügel-Rührers sah deutlich marmorierter aus. Es wurde hierbei nur 42% % der Trockenmasse als Oberlauf abgetrennt, beim Propeller-Rührer betrug der Anteil der abgetrennten Trockenmasse 50%.

Aufgrund der Analysen der Phasen kann gefolgert werden, dass mit dem Propeller-Rührer unerwünscht "Sonstiges" freigesetzt wird (die weiße Schicht über den Schalen ist beim Propeller-Rührer deutlich größer, (vermutlich handelt es sich um Stärke), dafür sind die Proteinverluste mit der Schalenfraktion leicht angestiegen. (Flügel 30%, Propeller 33%).

Es konnten darüber hinaus noch weitere vorteilhafte Verfahrensvarianten gefunden werden.

So verursacht eine hohe Alkohol-, insbesondere Ethanolkonzentration einen hohen Öl-Gehalt im "Globulin-Quark". Besonders vorteilhaft ist es im Schritt E) daher, wenn die Alkoholkonzentration kleiner als 20% ist, insbesondere 13 bis 18% beträgt, besonders vorzugsweise 15%,.

Eine zu lange Reaktionszeit bei ph 10 (über Nacht) verursacht ebenfalls hohe Ölgehalte im Globulin-Quark. Eher niedrigere Temperaturen, insbesondere unter 43°C, wirken sich vorteilhaft bei der Globulinfällung bzw. -abtrennung aus und bewirken höhere Proteingehalte im Quark. (Spalte K, Zeilen 34 und 35).

Als besonders vorteilhaft erscheinen weiter eine oder mehrere folgender weiterer Maßnahmen: Verwendung frischen Materials beim Pressen des Öls; kaltes Pressen des Öls; schonendes Rühren mit einem Flügel-Rührer (im Schritt C), dabei sollte das Material möglichst wenig geschert werden oder gar gemahlen werden; ein Alkohol-, insbesondere Ethanolgehalt von weniger als 20% erscheint besonders vorteilhaft, da sich ansonsten ein höherer Ölgehalt im Quark ergibt.

## Patentansprüche

1. Verfahren zur Gewinnung von Proteinen aus nativen Stoffgemengen, mit folgenden Schritten:
- Schritt A: Bereitstellen eines nativen Stoffgemenges aus Saaten mit harten, zerbrechbaren Schalen, insbesondere aus ganzen Leguminosensaaten, ganzen Sonnenblumenkernen (Saaten bzw. Früchte der *Helianthus annuus) oder Soyabohnen,* und/oder ganzen Saaten/Früchten von Kreuzblütengewächsen (Brassicaceae), insbesondere von Rapsfrüchten als Stoffgemenge aus den vollständigen Saaten oder aus bereits (teil-) entölten Saaten, insbesondere als Presskuchen, der bei einem Abpressen von Öl insbesondere mit einer Presse als Rückstand der Ölgewinnung verbleibt, "Vorstufe" bzw. auch "Ausgangsprodukt" genannt;
- Schritt B: sofern das Stoffgemenge aus Schritt A noch nicht zerkleinert ist: Zerkleinern des Stoffgemenges, wobei jedenfalls die Schalen aufgebrochen werden;
- Schritt C: Dispergieren des zerkleinerten Stoffgemenges aus Schritt A) oder B) mit Wasser, wobei auf einen Teil zerkleinertes Stoffgemenge vorzugsweise bis zu maximal 8, besonders vorzugsweise bis zu maximal 4 bis 5 oder 6 Teile Wasser zugegeben werden und wobei das Wasser und das zerkleinerte Stoffgemenge gerührt werden, so dass sich ein fließfähiger Brei bzw. eine Dispersion ergibt;
- Schritt D): Einstellen des pH-Wertes des Breis (I) aus Schritt C) in einen alkalischen Bereich pH > 9, 5;
- Schritt E): Zugeben des wasserlöslichen organischen Lösemittels Alkohol zu dem Brei aus Schritt D) im Anschluss an das Einstellen des pH-Wertes des Breis im Schritt D; derart, dass eine Alkoholkonzentration erreicht wird, die kleiner als 30% ist, um die Schalen vom Endosperm der Saaten/Früchte zu lösen;
- Schritt F): Abtrennen einer Feststoffphase, welche den überwiegenden Anteil der Schalen aufweist, vorzugsweise in einer Zentrifuge im Zentrifugalfeld;
- Schritt G): Verschieben des pH-Wertes des von Schalen befreiten Breis aus Schritt F) in den pH-Bereich von pH = 4,5 bis pH = 7,2; und
- Schritt H): Trennen des schalenfreien Breis, dessen pH-Wert in Schritt G) ins Saure verschoben worden ist, - vorzugweise in einer Zentrifuge, insbesondere in wenigstens einem Dekanter oder einem Separator in mehrere Phasen, wobei eine dieser Phasen eine Proteinkonzentratphase ist (Proteinquark), **dadurch gekennzeichnet, dass** die Proteinkonzentratphase (Proteinquark) unter Verwendung eines Mahltrockners zu einem Pulver getrocknet wird.

2. Verfahren nach Anspruch 1, **dadurch gekennzeichnet, dass** im Schritt H) folgende Phasentrennung in einem oder zwei Schritten, vorzugsweise in einer Zentrifuge, insbesondere in einem Dekanter oder Separator, vorgenommen wird:
- ölhaltige Phase mit Triglyceringehalt;
- wässrige Phase mit Albumingehalt; und
- die Proteinkonzentratphase (Proteinquark);

3. Verfahren nach Anspruch 1, **dadurch gekennzeichnet, dass** im Schritt H) folgende Phasentrennung in einem oder zwei Schritten, vorzugsweise in einer Zentrifuge, insbesondere in einem Dekanter oder Separator, in folgende zwei Wertstoffphasen vorgenommen wird:
- wässrige Phase mit Albumingehalt und Restölgehalt; und
- die Proteinkonzentratphase (Proteinquark);

4. Verfahren nach Anspruch 1, 2 oder 3, **dadurch gekennzeichnet, dass** eine Filtration der Wasserphase aus Schritt H) zur Albumin-Aufkonzentrierung erfolgt, um derart die Albuminphase als Wertstoff zu gewinnen.

5. Verfahren nach Anspruch 1, 2, 3 oder 4, **dadurch gekennzeichnet, dass** eine Proteinkonzentratphase gewonnen wird, die in der Farbeinstufungsskala "RAL" den Werten RAL 1015 oder RAL 1013 zuzuordnen ist.

6. Verfahren nach einem der vorstehenden Ansprüche, **dadurch gekennzeichnet, dass** als das Stoffgemenge/Ausgangsmaterial "kurz zuvor hergestelltes Zwischenprodukt" verarbeitet wird , d.h. nach der Vorstufe sind nicht mehr als 31 Tage vergangen.

7. Verfahren nach Anspruch 6, **dadurch gekennzeichnet, dass** als das Stoffgemenge/Ausgangsmaterial "frisches Zwischenprodukt" verarbeitet wird, d.h. nach der Vorstufe dürfen nicht mehr als 3 Tage vergangen sein, vorzugsweise sogar nur weniger als 48 Stunden oder 24 Stunden.

8. Verfahren nach Anspruch 7, **dadurch gekennzeichnet, dass** als das Stoffgemenge/Ausgangsmaterial "frisches Zwischenprodukt" verarbeitet wird, wobei nach der Vorstufe weniger als 12 h oder vorzugsweise weniger als 1 h vergangen sind/ist.

9. Verfahren nach einem der vorstehenden Ansprüche, **dadurch gekennzeichnet, dass** als das Stoffgemenge in Schritt A kalt gepressten Material, insbesondere ein kalt gepresster Rapspresskuchen verwendet wird, der bei einer Temperatur kleiner als 70°C, besonders vorzugsweise sogar kleiner als 60°C, gepresst worden ist.

10. Verfahren nach einem der vorstehenden Ansprüche, **dadurch gekennzeichnet, dass** im Schritt C) das Rühren für mehr als 30 min, insbesondere für mehr als 1 h erfolgt.

11. Verfahren nach einem der vorstehenden Ansprüche, **dadurch gekennzeichnet, dass** im Schritt D) das Rühren für mehr als 30 min, insbesondere für mehr als 1 h erfolgt.

12. Verfahren nach einem der vorstehenden Ansprüche, **dadurch gekennzeichnet, dass** die Abtrennungsschritte jeweils in einem 3-Phasendekanter oder in zumindest zwei Schritten in 2-Phasendekantern erfolgen.

13. Verfahren nach einem der vorhergehenden Ansprüche, **dadurch gekennzeichnet, dass** das wasserlösliche organische Lösemittel ein linearer aliphatischer Alkohol ist.

14. Verfahren nach einem der vorhergehenden Ansprüchen, **dadurch gekennzeichnet, dass** der Gehalt an wasserlöslichem organischen Lösemittel in dem Brei (I) nach dem Zugeben des wasserlöslichen alkoholischen Lösemittels in Schritt ii weniger als 45 Vol. %, vorzugsweise weniger als 30 Vol % und besonders vorzugsweise weniger als 15 Vol %, beträgt.

15. Verfahren nach einem der vorhergehenden Ansprüche, **dadurch gekennzeichnet, dass** vor Schritt H) ein Scheren des Breis erfolgt.

16. Verfahren nach einem der vorhergehenden Ansprüche, **dadurch gekennzeichnet, dass** vor Schritt H) und nach Schritt F) oder G) ein Scheren des weiter zu verarbeitenden Breis erfolgt.

17. Verfahren nach einem der vorhergehenden Ansprüche, **dadurch gekennzeichnet, dass** die Temperatur während sämtlicher Verfahrensschritte - wozu nicht das Pressen zur Erzeugen des Presskuchens gehört - kleiner 60 ° C liegt.

18. Verfahren nach einem der vorhergehenden Ansprüche, **dadurch gekennzeichnet, dass** die Temperatur im, Verfahrensschritt H) zwischen 20 und 30 °C liegt.

19. Verfahren nach einem der vorhergehenden Ansprüche, **dadurch gekennzeichnet, dass** die Temperatur während sämtlicher Verfahrensschritte - wozu nicht das dem Verfahren vorangehende Pressen zur Erzeugen eines Presskuchens als Ausgangsmaterial gehört- kleiner 50 ° C liegt.

20. Verfahren nach einem der vorhergehenden Ansprüche, **dadurch gekennzeichnet, dass** die Temperatur während sämtlicher Verfahrensschritte - wozu nicht das Pressen zur Erzeugen des Presskuchens gehört- zwischen 40° C und 50°C liegt.

21. Verfahren nach einem der vorhergehenden Ansprüche, **dadurch gekennzeichnet, dass** in einem oder mehreren der Schritte C) und D) und E) das Rühren mit einem Rührer derart erfolgt, dass eine im Wesentlichen laminare Strömung beim Rühren erzeugt wird.

22. Verfahren nach einem der vorhergehenden Ansprüche, **dadurch gekennzeichnet, dass** in einem oder mehreren der Schritte C) und D) und E) das Rühren mit einem Rührer derart erfolgt, dass keine produktschädigende Scherwirkung beim Rühren erzeugt wird.

23. Verfahren nach einem der vorhergehenden Ansprüche, **dadurch gekennzeichnet, dass** im Schritt E) das Zugeben des wasserlöslichen organischen Lösemittels Alkohol zu dem Brei aus Schritt D) im Anschluss an das Einstellen des pH-Wertes des Breis im Schritt D) derart erfolgt, dass die Alkoholkonzentration des Breis nach der Zugabe zwischen 10% und 20% liegt.

24. Verfahren nach einem der vorhergehenden Ansprüche, **dadurch gekennzeichnet, dass** im Schritt E) das Zugeben des wasserlöslichen organischen Lösemittels Alkohol zu dem Brei aus Schritt D) im Anschluss an das Einstellen des pH-Wertes des Breis im Schritt D) derart erfolgt, dass die Alkoholkonzentration des Breis nach der Zugabe zwischen 13% und 18% liegt.

## Claims

1. A method for obtaining proteins from native mixtures of materials, which comprises the following steps:
- Step A: Providing a native mixture of materials from seeds having hard fragmentable hulls, in particular from whole legume seeds, whole sunflower seeds (seeds or fruits of *Helianthus annuus) or soybeans,* and/or whole seeds/fruits of Brassicaceae, in particular of rape fruit as mixture of materials from whole seeds or from already (partly) deoiled seeds, in particular as press cake, which remains as a residue of oil extraction in the pressing of oil, in particular using a press, termed "preliminary stage" and/or also "starting product";
- Step B: If the mixture of materials from step A is not yet comminuted: comminuting the mixture of materials, wherein, in each case, the hulls are disintegrated;
- Step C: Dispersing the comminuted mixture of materials from step A) or B) with water, wherein, per one part of comminuted mixture of materials, preferably up to a maximum of 8, particularly preferably up to a maximum of 4 to 5, or 6 parts of water are added, and wherein the water and the comminuted mixture of materials are stirred, in such a manner that a free-flowing pulp and/or a dispersion is yielded;
- Step D): Adjusting the pH of the pulp (I) from step C) to an alkaline range pH > 9.5;
- Step E): Adding the water-soluble organic solvent alcohol to the pulp from step D) subsequently to adjusting the pH of the pulp in step D; in such a manner that an alcohol concentration is achieved which is less than 30%, in order to detach the hulls from the endosperm of the seeds/fruits;
- Step F): Separating off a solids phase which has the predominant fraction of the hulls, preferably in a centrifuge in the centrifugal field;
- Step G): Shifting the pH of the pulp from step F) that is freed from the hulls to the pH range of pH = 4.5 to pH = 7.2; and
- Step H): Separating the hull-free pulp, the pH of which has been shifted in step G) to the acid range, - preferably in a centrifuge, in particular in at least one decanter or a separator, into a plurality of phases, wherein one of these phases is a protein concentrate phase (protein curd), **characterized in that** the protein concentrate phase (protein curd) is dried to form a powder using a dryer-pulverizer.

2. The method as claimed in claim 1, **characterized in that**, in step H) the following phase separation is performed in one or two steps, preferably in a centrifuge, in particular in a decanter or separator:
- oily phase having a triglycerol content;
- aqueous phase having an albumin content; and
- the protein concentrate phase (protein curd).

3. The method as claimed in claim 1, **characterized in that** in step H), the following phase separation is carried out in one or two steps, preferably in a centrifuge, in particular in a decanter or separator, into the following two valuable material phases:
- aqueous phase having an albumin content and residual oil content; and
- the protein concentrate phase (protein curd).

4. The method as claimed in claim 1, 2 or 3, **characterized in that** the water phase from step H) is filtered for albumin concentration, in order in this manner to obtain the albumin phase as valuable material.

5. The method as claimed in claim 1, 2, 3 or 4, **characterized in that** a protein concentrate phase is obtained which is to be assigned the values RAL 1015 or RAL 1013 in the RAL color classification scale.

6. The method as claimed in any one of the preceding claims, **characterized in that**, at the mixture of materials/starting material, "intermediate product produced shortly before" is processed, that is to say, after the preliminary stage, no more than 31 days have passed.

7. The method as claimed in claim 6, **characterized in that**, as the mixture of materials/starting material, "fresh intermediate product" is processed, that is to say after the preliminary stage, no more than 3 days shall have passed, preferably even only fewer than 48 hours or 24 hours.

8. The method as claimed in claim 7, **characterized in that**, as the mixture of materials/starting material, "fresh intermediate product" is processed, wherein, after the preliminary stage, less than 12 h or preferably less than 1 h, have/has passed.

9. The method as claimed in any one of the preceding claims, **characterized in that**, as the mixture of materials in step A, cold-pressed material, in particular a cold-pressed rape press cake is used, which was pressed at a temperature below 70°C, particularly preferably even below 60°C.

10. The method as claimed in any one of the preceding claims, **characterized in that**, in step C), the stirring proceeds for more than 30 min, in particular for more than 1 h.

11. The method as claimed in any one of the preceding claims, **characterized in that**, in step D), the stirring is performed for more than 30 min, in particular for more than 1 h.

12. The method as claimed in any one of the preceding claims, **characterized in that** the separation steps are each performed in a three-phase decanter, or in at least two steps in two-phase decanters.

13. The method as claimed in any one of the preceding claims, **characterized in that** the water-soluble organic solvent is a linear aliphatic alcohol.

14. The method as claimed in any one of the preceding claims, **characterized in that** the content of water-soluble organic solvent in the pulp (I) after addition of the water-soluble alcoholic solvent in step ii is less than 45% by volume, preferably less than 30% by volume, and particularly preferably less than 15% by volume.

15. The method as claimed in any one of the preceding claims, **characterized in that**, before step H), the pulp is sheared.

16. The method as claimed in any one of the preceding claims, **characterized in that**, before step H) and after step F) or G), the pulp that is to be further processed is sheared.

17. The method as claimed in any one of the preceding claims, **characterized in that** the temperature during all of the method steps - which does not include the pressing for generating the press cake- is below 60°C.

18. The method as claimed in any one of the preceding claims, **characterized in that** the temperature in the method step H) is between 20 and 30°C.

19. The method as claimed in any one of the preceding claims, **characterized in that** the temperature during all of the method steps - which does not include the pressing for generating a press cake as starting material which precedes the method - is below 50°C.

20. The method as claimed in any one of the preceding claims, **characterized in that** the temperature during all of the method steps - which does not include the pressing for generating the press cake - is between 40°C and 50°C.

21. The method as claimed in any one of the preceding claims, **characterized in that**, in one or more of the steps C) and D) and E), the stirring is performed using a stirrer in such a manner that a substantially laminar flow is generated in the stirring.

22. The method as claimed in any one of the preceding claims, **characterized in that**, in one or more of the steps C) and D) and E), the stirring is performed using a stirrer in such a manner that no product-damaging shearing action is generated during the stirring.

23. The method as claimed in any one of the preceding claims, **characterized in that**, in step E), the addition of the water-soluble organic solvent alcohol to the pulp from step D) is performed subsequently to the adjusting of the pH of the pulp in step D) in such a manner that the alcohol concentration of the pulp after the addition is between 10% and 20%.

24. The method as claimed in any one of the preceding claims, **characterized in that**, in step E), the addition of the water-soluble organic solvent alcohol to the pulp from step D) is performed subsequently to the adjusting of the pH of the pulp in step D) in such a manner that the alcohol concentration of the pulp after the addition is between 13% and 18%.

## Revendications

1. Procédé d'obtention de protéines à partir de mélanges de matières natives comprenant les étapes suivantes consistant à :
- Etape A : préparer un mélange de matières natives à partir de semences ayant des peaux dures cassantes, en particulier de semences de légumineuses entières, de graines de tournesol entières (semences ou fruits de *Helianthus annus*) ou de graines de soja et/ou de semences / fruits entiers de plantes crucifères (Brassicaceae) en particulier de fruits de colza en tant que mélange de matières, provenant de semences totales et/ou de semences déjà le cas échéant partiellement déshuilées, en particulier sous la forme de tourteaux de pression obtenus en tant que résidus lors de l'obtention d'huile par pression en particulier avec une presse, nommé « étape préliminaire » ou également « produit de départ »,
- Etape B : dans la mesure où le mélange de matières provenant de l'étape A n'a pas encore été concassé, concasser ce mélange de matière, les peaux étant toujours rompues,
- Etape C : disperser le mélange de matières concassé provenant de l'étape A ou de l'étape B avec de l'eau, dans laquelle pour une partie du mélange de matières concassé ou ajouté de préférence jusqu'à un maximum de huit, de façon particulièrement préférentielle jusqu'à un maximum de 4 à 5 ou 6 parties d'eau, et on agite l'eau et le mélange de matières concassé de façon à obtenir une dispersion ou une bouillie fluide,
- Etape D : régler le pH- de la bouillie (I) provenant de l'étape (C) dans une plage alcaline de pH >9,5,
- Etape E : ajouter un solvant organique alcoolique soluble dans l'eau à la bouillie provenant de l'étape D à la suite du réglage de la valeur du pH de la bouillie dans l'étape D de façon à atteindre une concentration en alcool inférieure à 30% pour permettre de débarrasser par dissolution les peaux des endospermes des semences /Fruits,
- Etape F : séparer une phase solide qui renferme la plus grande proportion des peaux de préférence dans une centrifugeuse dans un champ de centrifugation,
- Etape G : décaler la valeur du pH de la bouillie libérée des peaux provenant de l'étape F dans une plage de pH de pH= 4,5 à pH= 7,2, et
- Etape H : séparer la bouillie exempte de peaux dont la valeur du pH a été décalée dans la plage acide lors de l'étape G, de préférence dans une centrifugeuse, en particulier dans au moins un décanteur ou un séparateur en plusieurs phases, l'une de ces phases étant une phase de concentrat de protéines (fromage de protéines),
**caractérisé en ce que**
la phase de concentrât de protéines (fromage de protéines) est séchée de façon à obtenir une poudre en utilisant un sécheur broyeur.

2. Procédé conforme à la revendication 1,
**caractérisé en ce que**
dans l'étape H), on effectue la séparation des phases suivante en une ou deux étapes, de préférence dans une centrifugeuse, en particulier dans un décanteur ou un séparateur :
- phase huileuse renfermant des triglycérides,
- phase aqueuse renfermant de l'albumine et,
- la phase de concentrat de protéines (fromage de protéines).

3. Procédé conforme à la revendication 1,
**caractérisé en ce que**
dans l'étape H) on effectue la séparation de phases suivantes en une ou deux étapes, de préférence dans une centrifugeuse, en particulier dans un décanteur ou un séparateur, de façon à obtenir les deux phases de produit valorisable suivante :
- phase aqueuse renfermant de l'albumine et de l'huile résiduelle, et
- la phase de concentrat de protéines (fromage de protéines).

4. Procédé conforme à la revendication 1, 2 ou 3,
**caractérisé en ce que**
l'on effectue une filtration de la phase aqueuse provenant de l'étape H) de façon à la concentrer en albumine pour obtenir ainsi une phase d'albumine en tant que produit valorisable.

5. Procédé conforme à la revendication 1, 2, 3 ou 4,
**caractérisé en ce que**
l'on obtient une phase de concentrat de protéines qui, dans l'échelle de classement de couleur "RAL" est associée aux valeurs RAL 1015 ou RAL 1013.

6. Procédé conforme à l'une des revendications précédentes,
**caractérisé en ce que**
en tant que mélange de matières / matériau de départ on traite un « produit intermédiaire obtenu peu auparavant » ce qui signifie que, après l'étape préalable pas plus de 31 jours se sont écoulés.

7. Procédé conforme à la revendication 6,
**caractérisé en ce qu'**
en tant que mélange de matières / matériau de départ on traite un "produit intermédiaire frais" ce qui signifie que, après l'étape préalable, pas plus de 3 jours doivent s'être écoulés, de préférence uniquement moins de 48 heures ou 24 heures.

8. Procédé conforme à la revendication 7,
**caractérisé en ce qu'**
en tant que mélange de matières / matériau de départ on traite un « produit intermédiaire frais » après l'étape intermédiaire, moins de 12 h ou de préférence moins d'1 heure s'étant écoulé.

9. Procédé conforme à l'une des revendications précédentes,
**caractérisé en ce que**
en tant que mélange de matières on utilise dans l'étape A) des matières pressées à froid, en particulier un tourteau de colza pressé à froid qui a été pressé à une température inférieure à 70°C et de façon particulièrement préférentielle même intérieure à 60°C.

10. Procédé conforme à l'une des revendications précédentes,
**caractérisé en ce que**
lors de l'étape C) l'agitation est effectuée pendant plus de 30 minutes, et en particulier pendant plus d'une heure.

11. Procédé conforme à l'une des revendications précédentes,
**caractérisé en ce que**
lors de l'étape D) l'agitation est effectuée pendant plus de 30 minutes, en particulier pendant plus d'une heure.

12. Procédé conforme à l'une des revendications précédentes,
**caractérisé en ce que**
les étapes de séparation sont respectivement effectuées dans un décanteur trois phases ou en au moins deux étapes dans un décanteur deux phases.

13. Procédé conforme à l'une des revendications précédentes,
**caractérisé en ce que**
le solvant organique soluble dans l'eau est un alcool alphifatique linéaire.

14. Procédé conforme à l'une des revendications précédentes,
**caractérisé en ce que**
la teneur en solvant organique soluble dans l'eau dans la bouillie (I) après l'addition de solvant alcoolique soluble dans l'eau dans l'étape ii est inférieure à 45% en volume de préférence inférieure à 30% en volume et de façon particulièrement préférentielle inférieure à 15% en volume.

15. Procédé conforme à l'une des revendications précédentes,
**caractérisé en ce que**
avant l'étape H) on effectue un cisaillement de la bouillie.

16. Procédé conforme à l'une des revendications précédentes,
**caractérisé en ce que**
avant l'étape H) et après l'étape F) ou G) on effectue un cisaillement de la bouillie devant subir un traitement ultérieur.

17. Procédé conforme à l'une des revendications précédentes,
**caractérisé en ce que**
la température pendant la totalité des étapes du procédé - dont ne fait pas partie le pressage permettant d'obtenir le tourteau - est inférieur à 60°C.

18. Procédé conforme à l'une des revendications précédentes,
**caractérisé en ce que**
lors de l'étape H) la température est comprise entre 20 et 30°C.

19. Procédé conforme à l'une des revendications précédentes,
**caractérisé en ce que**
la température pendant la totalité des étapes de procédé - dont ne fait pas partie le pressage préalable au procédé permettant d'obtenir un tourteau en tant que matériau de départ - est inférieure à 50°C.

20. Procédé conforme à l'une des revendications précédentes,
**caractérisé en ce que**
la température pendant la totalité des étapes du procédé - dont ne fait pas partie le pressage permettant d'obtenir le tourteau - est comprise entre 40°C et 50°C.

21. Procédé conforme à l'une des revendications précédentes,
**caractérisé en ce que**
lors d'une ou de plusieurs des étapes C) et D) et E), l'agitation est effectuée avec un agitateur de façon à obtenir lors de l'agitation un écoulement essentiellement laminaire.

22. Procédé conforme à l'une des revendications précédentes,
**caractérisé en ce que**
lors d'une ou de plusieurs des étapes C), D) et E), l'agitation est effectuée avec un agitateur de façon à ne pas obtenir lors de l'agitation un effet de cisaillement endommageant le produit.

23. Procédé conforme à l'une des revendications précédentes,
**caractérisé en ce que**
lors de l'étape E), l'addition du solvant organique alcoolique soluble dans l'eau à la bouillie provenant de l'étape D) à la suite du réglage de la valeur du pH de la bouillie lors de l'étape D) est effectuée de sorte que la concentration en alcool de la bouillie après l'addition soit comprise entre 10% et 20%.

24. Procédé conforme à l'une des revendications précédentes,
**caractérisé en ce que**
lors de l'étape E) l'addition du solvant organique alcoolique soluble dans l'eau à la bouillie provenant de l'étape D) à la suite du réglage d la valeur du pH de la bouillie dans l'étape D) est effectuée de sorte que la concentration en alcool de la bouillie après l'addition soit comprise entre 13% et 18%.
